# EUROPEAN PATENT APPLICATION

(11) **EP 4 706 690 A1**
(43) Date of publication of application: **11.03.2026**
(21) Application number: 24810510.8
(22) Date of filing: 25.07.2024
(51) Int. Cl.: A61K 51/08, A61K 49/04, A61K 49/14, A61K 49/00, A61K 31/6615, A61K 47/64, A61K 41/00, A61P 35/00, A61K 103/00, A61K 103/10, A61K 103/30, A61K 103/20

(54) **PHYTIC ACID-BASED TERNARY COMPLEX BIONIC NANO MATERIAL, AND PREPARATION METHOD AND USE THEREOF**

(30) Priority: 25.05.2023 CN 202310595063
(71) Applicant: The Second Hospital of Tian Jin Medical University, Tianjin 300211 (CN)
(72) Inventor: ZHAO, Yang, Tianjin 300211 (CN); DUAN, Dengyi, Tianjin 300211 (CN); HAN, Gang, Nanjing, Jiangsu 210037 (CN); PENG, Jing, Tianjin 300211 (CN)
(74) Representative: Swea IP Law AB
(86) International application number: PCT/CN2024/107540
(87) International publication number: WO 2024/240275

(57) **Abstract**

The present application relates to the field of biological medicines, in particular to a phytic acid-based ternary complex bionic nano material. The ternary complex bionic nano material is composed of phytic acid, metal ions and a protein/polypeptide. The ternary complex is formed by connecting the protein/polypeptide with phytic acid using metal cations as a cation bridge. According to the application, the phytic acid ternary complex having different protein/polypeptide groups is synthesized by using various metal ions as bridges. A novel nano diagnosis and treatment probe having high biocompatibility is constructed, which can be reassembled and gathered for a long time at a tumor part, and has coordination-loaded imaging/therapeutic metal ions. The functions of long-acting tumor imaging, treatment or integrated diagnosis and treatment can be achieved, and the solution can be expected to be used as a novel bionic nano material in the field of biomedicine. In addition, the preparation process of the nano material is simple, and industrial production is easy to implement.

## Description

The present disclosure claims priority to Chinese Patent Application No. 202310595063.5 filed with the China National Intellectual Property Administration on May 25, 2023 and entitled "PHYTIC ACID-BASED TERNARY COMPLEX BIOMIMETIC NANOMATERIAL, AND PREPARATION METHOD AND USE THEREOF", which is incorporated herein by reference in its entirety.

### TECHNICAL FIELD

The present disclosure relates to the preparation of a nanomaterial, in particular relates to the preparation and use of a phytic acid-based ternary complex biomimetic nanomaterial.

### BACKGROUND

As tumor therapy progresses into the era of "precision medicine", the delivery of imaging and therapeutic drugs via biomimetic nanomaterials holds broad application prospects in the accurate diagnosis and therapy of tumors. However, insufficient tumor accumulation time and poor biocompatibility are the main limitations that hinder the further development and clinical translation of biomimetic nanomaterials. Strategies such as modification with targeting molecules and construction of stimuli-responsive probes can enhance the accumulation of nanoparticles in tumors, prolong the retention time of nanoparticles in tumors, and improve therapeutic efficacy. However, the retention time of these nanoparticles in tumors is still not ideal. This is because they can re-enter the bloodstream or diffuse into surrounding tissues, failing to provide long-term and real-time tumor monitoring and therapy. In addition, the biocompatibility of the nanoparticles is not ideal, which may lead to long-term toxicity due to the retention of drugs in the body. Therefore, it is necessary to develop a biocompatible therapeutic probe that resides in a tumor site for a long time to break the spatiotemporal limitations, thereby achieving precise therapy and tumor monitoring.

Phytic acid (PA), also known as inositol hexaphosphate, is a type of vitamin B that is widely found in plants such as legumes, cereals, dried fruits, vegetables and fruits. It is also present in most mammalian cells, which contributes to its good biocompatibility. PA has a myo-inositol hexaphosphate structure, with a six-carbon ring molecular conformation that exhibits asymmetry. The PA molecule contains six phosphate groups and twelve hydroxyl groups, which endow it with a strong chelating capability. PA also exhibits inherent anticancer ability, inhibiting the growth of various cancer cells, such as those of leukemia, prostate cancer, breast cancer, liver cancer, colon cancer and skin cancer.

The molecular structure of PA confers very strong chelating chemical properties. PA carries a negative charge over a wide pH range, which allows it chelates with many positively charged divalent or multivalent metal ions (such as Mg²⁺, Fe²⁺ and Cd²⁺). The resulting complexes have a very low solubility, and are called phytates. Some phytates are widely used in biomedical applications. For example, technetium [99mTc] phytate injection serves as a Positron Emission Tomography (PET) contrast agent for visualizing the liver, spleen and bone marrow. However, the low solubility of many phytates limits their potential for further biomedical applications.

### SUMMARY

Aiming at problems of poor biocompatibility, insufficient accumulation time, and safety concerns associated with *in vivo* accumulation of constructed biomimetic nanomaterials in the prior art, the present disclosure provides a biomimetic nanomaterial that has high biocompatibility, possesses multiple biological functions such as tumor targeting, tumor therapy and magnetic resonance imaging, and can maintain a long retention time at a tumor site. The use of the biomimetic nanomaterial enables continuous radiotherapy and chemotherapy for tumors while allowing for long-term imaging monitoring of changes in the tumor treatment.

In order to solve the above technical problems, the present disclosure proposes a phytic acid (PA) -based ternary complex biomimetic nanomaterial. The biomimetic nanomaterial is a ternary complex composed of PA, a metal ion and a protein/polypeptide, where a molar ratio of the PA to the metal ion is 1 : (1-6), and a molar ratio of the protein/polypeptide to the PA is 1 : 25; and the ternary complex is formed by connecting the protein/polypeptide with the PA using a metal cation as a cation bridge.

Further, in the PA-based ternary complex biomimetic nanomaterial of the present disclosure,
the metal ion is a multivalent metal cation.

The protein/polypeptide is a negatively charged protein/polypeptide at a pH value of 7-10.

The selection of the multivalent metal cation includes several scenarios shown in Table 1.

**Table 1 Selection of the multivalent metal cation for different imaging and therapeutic applications**

| Application | Multivalent metal cation |
|---|---|
| For preparing a contrast agent for magnetic resonance imaging (MRI) | Cr³⁺, Co²⁺, Mn²⁺, Fe²⁺, Fe³⁺, Cu²⁺, Cu³⁺, La²⁺, Gd³⁺, Ce³⁺, Tb³⁺, Pr³⁺, Dy³⁺, Nd³⁺, Ho³⁺, Pm³⁺, Er³⁺, Sm³⁺, Tm³⁺, Eu³⁺, Yb³⁺ and Lu³⁺ |
| For preparing a contrast agent for computed tomography (CT) imaging | Yb³⁺, Lu³⁺, Bi²⁺, Hf⁴⁺, Re⁴⁺, W⁴⁺ and Ta⁵⁺ |
| For preparing a contrast agent for radionuclide imaging | ¹⁷⁷Lu³⁺, ⁶⁸Ga³⁺, ^{99m}Tc⁷⁺, Sm³⁺, ¹¹¹In³⁺ and ⁶²Cu²⁺ |
| For preparing a contrast agent for optical imaging | Yb³⁺, Lu³⁺, Er³⁺ and Nd³⁺ |
| For preparing a drug for chemotherapy | Pt²⁺, Ru³⁺ and Os²⁺ |
| For preparing a drug for radiotherapy | ¹⁷⁷Lu³⁺, ²²³Ra²⁺, ⁸⁹Sr²⁺, ⁶⁰Co²⁺, ¹⁹²Ir³⁺ and ¹⁸²Ta⁵⁺ |
| For preparing a drug for photothermal therapy | Re⁴⁺, W⁴⁺ and Bi²⁺ |

Moreover, the present disclosure further provides two preparation methods for the above PA-based ternary complex biomimetic nanomaterial.

Preparation method I includes the steps of:
S1-1: mixing PA with a metal ion solution at a molar ratio of PA to metal ion of 1 : (1-6), and stirring until uniform to obtain mixed solution A;
S1-2: adding a protein/polypeptide solution to the mixed solution A at a molar ratio of protein/polypeptide to PA of 1 : 25, and adjusting a pH value of a resulting solution to 7-10 to obtain mixed solution B; and
S1-3: centrifuging the mixed solution B, remove a resulting pellet, and subjecting a resulting supernatant to repeated ultrafiltration/dialysis to obtain a ternary complex solution, which is the biomimetic nanomaterial.

Preparation method II includes the steps of:
S2-1: providing a PA solution, adjusting a pH value of the PA solution to 7-10, adding a protein/polypeptide solution to the pH-adjusted PA solution at a molar ratio of protein/polypeptide to PA of 1 : 25, and stirring until uniform to obtain mixed solution C;
S2-2: adding a metal ion solution to the mixed solution C at a molar ratio of metal ion to PA of 3 : 1, and stirring until uniform to obtain mixed solution D, where the metal ion in the metal ion solution is a metal ion that produces a precipitate after mixed with the PA solution; and
S2-3: centrifuging the mixed solution D to remove the precipitate, and subjecting a resulting supernatant to repeated ultrafiltration/dialysis to obtain a ternary complex solution, which is the biomimetic nanomaterial.

The specific selection of preparation method I or preparation method II is mainly based on whether a precipitate is produced when the selected metal ion solution is mixed with the PA solution at a molar ratio of PA to metal ion of 1 : (1-6). If no precipitate is produced, both preparation method I and preparation method II can be adopted; and if a precipitate is produced, preparation method II is selected.

Further, in the above preparation method II, the metal ion is preferably one or more of Gd³⁺, Bi³⁺ and ²²³Ra²⁺.

The present disclosure further proposes that the above PA-based ternary complex biomimetic nanomaterial can be used for the preparation of a contrast agent for one or more of MRI, CT imaging, radionuclide imaging and optical imaging, and for the preparation of a drug for chemotherapy, radiotherapy or photothermal therapy. The selection of the multivalent metal cation is shown in Table 1.

Compared with the conventional technology, embodiments of the present disclosure have the following beneficial effects.

The present disclosure proposes a construction strategy for a PA-based multifunctional biomimetic nanomaterial. After food is ingested by the human body, both the protein and PA are negatively charged under the basic conditions of the intestinal tract. In this case, a PA-metal cation-protein ternary complex can be formed using a metal cation as a bridge. The addition of the protein can endow the complex with advantages such as good biocompatibility and biodegradability, low antigenicity, high stability and drug-loading capacity. By means of simulating the formation of ternary complexes in the intestinal tract, the biomimetically synthesized ternary complex biomimetic nanomaterial in the present disclosure not only has high biocompatibility, but also exhibits multiple biological functions such as tumor targeting, tumor therapy and magnetic resonance imaging. The prepared biomimetic nanomaterial can undergo *in-situ* reassembly at the tumor site to maintain a long retention time, thereby achieving long-term MRI monitoring and chemo-radiotherapy effects on tumors. Moreover, the biomimetic nanomaterial has excellent biocompatibility, and may significantly reduce the toxicity of chemo-radiotherapeutic drugs, demonstrating good clinical translation value. In addition, the preparation method for the biomimetic nanomaterial is simple, easy to operate, and low in cost. The nanomaterial is easy for industrial production and is expected to be used as a novel nanotheranostic probe for applications in the biomedical field.

### BRIEF DESCRIPTION OF THE DRAWINGS

FIG. 1 shows the construction principle of the PA-based ternary complex biomimetic nanomaterials of the present disclosure;
FIG. 2 shows the transmission electron micrograph of PA-Mn-BSA (PA : Mn = 1 : 3) prepared in Example 1;
FIG. 3 shows the transmission electron micrograph of PA-Gd-BSA prepared in Example 2;
FIG. 4 shows the transmission electron micrograph of PA-Mn/Pt-BSA (PA : Mn : Pt = 1 : 1 : 2) prepared in Example 4;
FIG. 5 shows the photograph and hydrodynamic particle size distribution plot of PA-Bi-BSA prepared in Example 3;
FIG. 6 shows the photograph and hydrodynamic particle size distribution plot of PA-Mn/²²³Ra-BSA prepared in Example 5;
FIG. 7 shows the photograph and hydrodynamic particle size distribution plot of PA-Mn/Pt-RGD prepared in Example 6;
FIG. 8 shows the *in vitro* magnetic resonance images of PA-Mn-BSA (PA : Mn = 1 : 3) and PA-Mn/Pt-BSA (PA : Mn : Pt = 1 : 1 : 2) prepared in Examples 1 and 4;
FIG. 9 shows the *in vitro* magnetic resonance image of PA-Gd-BSA prepared in Example 2;
FIG. 10 shows the *in vitro* magnetic resonance image of PA-Mn/²²³Ra-BSA prepared in Example 5;
FIG. 11 shows the *in vitro* magnetic resonance image of PA-Mn/Pt-RGD prepared in Example 6;
FIG. 12 shows the *in vitro* CT image of PA-Bi-BSA prepared in Example 3;
FIG. 13 shows the particle size stability of PA-Mn-BSA (PA : Mn = 1 : 3) and PA-Mn/Pt-BSA (PA : Mn : Pt = 1 : 1 : 2) prepared in Examples 1 and 4 in phosphate-buffered saline (PBS) and fetal bovine serum (FBS);
FIG. 14 shows the reassembly response of PA-Mn-BSA (PA : Mn = 1 : 3) prepared in Example 1 to the acidic environment, in which (a) shows the photograph of PA-Mn-BSA (PA : Mn = 1 : 3) at pH 7.4 and 5.0, (b) shows the particle size change of PA-Mn-BSA (PA : Mn = 1 : 3) at pH 7.4 and 5.0, and (c) shows the transmission electron micrograph of the PA-Mn-BSA (PA : Mn = 1 : 3) at pH 5.0;
FIG. 15 shows the killing effect of PA-Mn-BSA (PA : Mn = 1 : 3) of Example 1, PA-Mn/Pt-BSA (PA : Mn : Pt = 1 : 1 : 2) of Example 4, and the chemotherapeutic drug cisplatin (CDDP) on prostate cancer PC-3 cells;
FIG. 16 shows the killing effect of PA-Mn/²²³Ra-BSA of Example 5 on prostate cancer PC-3 cells;
FIG. 17 shows the killing effect of PA-Mn/Pt-RGD of Example 6 on prostate cancer PC-3 cells;
FIG. 18 shows the *in vivo* magnetic resonance imaging effect of PA-Mn-BSA (PA : Mn = 1 : 3) of Example 1 and PA-Mn/Pt-BSA (PA : Mn : Pt = 1 : 1 : 2) of Example 4, in which (a) shows the *in vivo* magnetic resonance imaging effect of PA-Mn-BSA (PA : Mn = 1 : 3), and (b) shows the *in vivo* magnetic resonance imaging effect of PA-Mn/Pt-BSA (PA : Mn : Pt = 1 : 1 : 2);
FIG. 19 shows the *in vivo* tumor inhibitory effects of four drugs, in which (a) the photographs of tumors dissected from PC-3 tumor-bearing mice 14 days after treatment with PBS, PA-Mn-BSA (PA : Mn = 1 : 3) of Example 1, CDDP, and PA-Mn/Pt-BSA (PA : Mn : Pt = 1 : 1 : 2) of Example 4, and (b) shows the tumor growth curves of PC-3 tumor-bearing mice treated with PBS, PA-Mn-BSA (PA : Mn = 1 : 3) of Example 1, CDDP, and PA-Mn/Pt-BSA (PA : Mn : Pt = 1 : 1 : 2) of Example 4;
FIG. 20 shows the comparison of Pt content in tumors 14 days after treatment with CDDP and PA-Mn/Pt-BSA (PA : Mn : Pt = 1 : 1 : 2) of Example 4.
FIG. 21 shows the photographs of tumors dissected from PC-3 tumor-bearing mice 10 days after treatment with PBS, PA-Mn-BSA (PA : Mn = 1 : 3) of Example 1, ²²³RaCl₂, and PA-Mn/²²³Ra-BSA of Example 5; and
FIG. 22 shows the biochemical analysis results of mice after injection with PA-Mn/Pt-BSA (PA : Mn : Pt = 1 : 1 : 2) of Example 4, in which (a) shows the level of aspartate transaminase (AST), (b) shows the level of alanine transaminase (ALT), (c) shows the level of blood urea nitrogen (BUN), and (d) shows the level of creatinine (CRE).

### DETAILED DESCRIPTION OF THE EMBODIMENTS

The design concept of the preparation method for a biomimetic nanomaterial proposed in the present disclosure is as follows: Using a biomimetic synthesis strategy, a highly biocompatible multifunctional biomimetic nanomaterial is prepared for use in the biomedical field. The biomimetic nanomaterial coordinates and loads a variety of imaging/therapeutic drugs to meet the requirements of diverse biomedical applications. According to the preparation method of the present disclosure, by means of simulating the formation of PA-based ternary complexes in the intestinal tract, different protein/polypeptide-based PA-based ternary complexes are biomimetically synthesized using various metal ions such as manganese and gadolinium as bridges. A highly biocompatible nanotheranostic probe is constructed, which can achieve long-term accumulation at the tumor site, possesses multiple functions such as tumor imaging and therapy, and is expected to be used as a novel biomimetic nanomaterial for use in the field of biomedicine. Moreover, the preparation process of the theranostic agent of the present disclosure is simple and easy for industrial production.

The present disclosure is further described below with reference to the accompanying drawings and specific examples, but the following examples are by no means intended to limit the present disclosure.

The biomimetic nanomaterial of the present disclosure means that the synthesized material is a nanoparticle that can be used in tumor imaging, tumor therapy or integrated theranostics.

The biomimetic nanomaterial of the present disclosure is a PA-based ternary complex, which includes PA, a metal ion coordinately chelated with PA, and a protein/polypeptide, where a molar ratio of the PA to the metal ion is 1 : (1-6), and a molar ratio of the protein/polypeptide to the PA is 1 : 25; the ternary complex is formed by connecting the protein/polypeptide with the PA using a metal cation as a cation bridge, serving as a biomimetic nanomaterial for application in tumor imaging, diagnosis and therapy.

In the biomimetic nanomaterial of the present disclosure, the metal ion is a multivalent metal cation. The protein/polypeptide is a negatively charged protein/polypeptide at a pH value of 7-10. In the present disclosure, the protein/polypeptide refers to a protein or polypeptide.

The present disclosure provides two methods for preparing the above biomimetic nanomaterial.

A first method includes the steps of: mixing PA and a metal ion solution at a molar ratio of PA to metal ion of 1 : (1-6), and stirring the mixture uniformly to obtain mixed solution A; adding a protein/polypeptide solution to the mixed solution A at a molar ratio of protein/polypeptide to PA of 1 : 25, and adjusting a pH value of the solution to 7-10 to obtain mixed solution B; and centrifuging mixed solution B to remove the precipitate, and repeatedly subjecting the supernatant to ultrafiltration/dialysis to obtain a ternary complex solution, which is the biomimetic nanomaterial.

A second method includes the steps of: providing a PA solution, adjusting a pH value of the PA solution to 7-10, adding a protein/polypeptide solution to the pH-adjusted PA solutionat a molar ratio of protein/polypeptide to PA of 1 : 25, and stirring the mixture uniformly to obtain mixed solution C; adding a metal ion solution to the mixed solution C at a molar ratio of metal ion to PA of 3 : 1, and stirring the mixture uniformly to obtain mixed solution D, where the metal ion in the metal ion solution is a metal ion that produces a precipitate after mixed with the PA solution; and centrifuging mixed solution D to remove the precipitate, and repeatedly subjecting the supernatant to ultrafiltration/dialysis to obtain a ternary complex solution, which is the biomimetic nanomaterial.

In the present disclosure, the ultrafiltration/dialysis refers to ultrafiltration or dialysis.

In the preparation process of the biomimetic nanomaterial of the present disclosure, the multivalent metal cation includes one or more of the elements listed in Table 1. Those skilled in the art can apply the multivalent metal cations to different imaging and therapy regimens depending on the physicochemical properties of the elements used. The preferred metal cations for magnetic resonance imaging (MRI) are Fe²⁺, Fe³⁺, Mn²⁺ and Gd³⁺.

In the preparation process of the biomimetic nanomaterial of the present disclosure, the preferred molar ratio of the PA to the multivalent metal cation is 1 : 3; the protein/polypeptide may be any one of proteins/polypeptides, such as common bovine serum albumin (BSA), human serum albumin (HSA), targeting protein transferrin (Tf), phycocyanin with photothermal properties, and targeting peptide Arg-Gly-Asp (RGD).

In the preparation process of the biomimetic nanomaterial of the present disclosure, the pH is adjusted within a range. The pH value is determined on the basis of the isoelectric point of the protein/polypeptide, and the pH range falls within the selected 7-10. For the convenience of biomedical applications, the preferred pH value is 7-7.5 within the allowable pH range.

The biomimetic nanomaterial of the present disclosure can be designed as biomimetic nanomaterials of different functions for use in tumor imaging diagnosis, tumor therapy and integrated theranostics. The use for tumor imaging includes, but is not limited to, the following imaging modalities: MRI, CT imaging, radionuclide imaging, optical imaging, etc. The use for tumor therapy includes, but is not limited to, the following therapeutic modalities: chemotherapy, radiotherapy, photothermal therapy, etc.

The present disclosure is further described in detail below with reference to examples. It should also be understood that the following examples are only for the purpose of further describing the present disclosure and are not to be construed as limiting the scope of protection of the present disclosure, and that some non-essential improvements and adjustments made by those skilled in the art on the basis of the above contents of the present disclosure are within the scope of protection of the present disclosure.

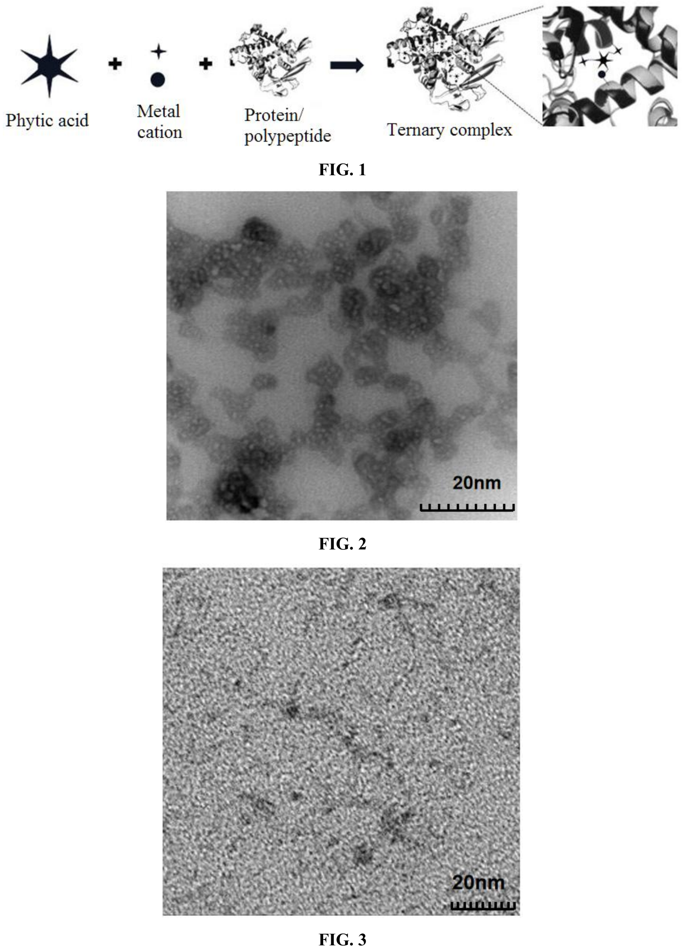

The construction of the PA-based ternary complex biomimetic nanomaterial in the present disclosure is achieved by connecting the protein/polypeptide and the PA with a metal cation as a bridge, thereby forming a PA-metal-protein/polypeptide ternary complex nanoparticle, the principle of which is shown in FIG. 1. When PA, a metal cation and a protein/polypeptide are present together, they can form a ternary complex under specific conditions by connecting negatively charged PA and protein/polypeptide with a metal cation as a cation bridge.

### Example 1

### (1) PA-Mn-BSA biomimetic nanomaterials were prepared according to method I, with specific steps as follows:

Firstly, 125 µL of a 0.1 M PA solution was added to a reaction flask containing 9 ml of double-distilled water (ddH₂O). According to different ratios of PA : Mn, including 1 : 1, 1 : 2, 1 : 3, 1 : 4, 1 : 5 and 1 : 6, 125 µL, 250 µL, 375 µL, 500 µL, 625 µL and 750 µL of 0.1 M MnCl₂ solutions were correspondingly added dropwise. The mixture was stirred uniformly. Then, 500 µL of a 1 mM BSA solution was added to the reaction flask, and the pH of the solution was quickly adjusted to 7 with a 1 M NaOH solution. The mixture was stirred at room temperature for 10 minutes. The solution was collected, and then centrifuged at 10,000 rpm for 20 minutes. The supernatant was transferred to an ultrafiltration tube and subjected to repeated ultrafiltration three times at 4000 rpm to remove impurities, thereby obtaining six PA-Mn-BSA solution samples.

### (2) Determination of the relaxivity of PA-Mn-BSA prepared in different molar ratios

This experiment was performed to screen for the preferred PA-to-metal ion ratio of PA : Mn for the PA-based ternary complex. Taking the above six PA-Mn-BSA biomimetic nanomaterials prepared in different molar ratios of PA : Mn as examples, the relaxivity of each PA-Mn-BSA sample was determined to identify the preferred PA-to-metal ion ratio of the constructed PA-based ternary complex biomimetic nanomaterials for magnetic resonance imaging.

The biomimetic nanomaterial samples with different concentration ratios of PA : Mn were each placed on the sample holder of a 1.5 T nuclear magnetic resonance spectrometer for T1 value measurement. On the basis of T1 values of the samples at different concentrations, linear regression was performed with concentration (C) as the abscissa and T1 value as the ordinate to construct a fitting curve, and then the longitudinal relaxivity (r1) values of the samples were calculated.

The results are shown in Table 2. When PA : Mn = 1 : 3, the PA-Mn-BSA nanoparticles had a high relaxivity and a high Mn ion loading efficiency. The ratio of 1 : 4 resulted in a higher relaxivity, but the Mn ion loading effect was significantly reduced. Therefore, the preferred ratio of PA to the metal ion in the PA-based ternary complex is 1 : 3.

**Table 2 Relaxivity of PA-Mn-BSA samples at different ratios**

| PA/Mn | r1 (mM⁻¹S⁻¹) | Mn (yield%) |
|---|---|---|
| 1 : 1 | 8.32 | 59.07 |
| 1 : 2 | 5.76 | 50.14 |
| 1 : 3 | 14.72 | 23.60 |
| 1 : 4 | 32.16 | 1.31 |
| 1 : 5 | 18.79 | 1.37 |
| 1 : 6 | 5.3 | 0.96 |

### Example 2

On the basis of the construction principle of the PA-based ternary complex biomimetic nanomaterial shown in FIG. 1, a protein and PA were connected using a paramagnetic substance Gd³⁺ as a cation bridge, thereby forming a PA-Gd-BSA ternary complex nanoparticle.

A PA-Gd-BSA nanoprobe was prepared according to the procedures of method II, with specific steps as follows: 125 µL of a 0.1 M PA solution was added to a reaction flask containing 9 ml of ddH₂O. The pH of the solution was adjusted to 7 with a 1 M NaOH solution. After adding 500 µL of a 1 mM BSA solution to the reaction flask, 375 µL of a 0.1 M MnCl₂ solution was added dropwise. The mixture was stirred at room temperature for reaction for 10 minutes. The solution was collected, and then centrifuged at 10,000 rpm for 20 minutes. The supernatant was transferred to an ultrafiltration tube and subjected to repeated ultrafiltration three times at 4000 rpm to remove impurities, thereby obtaining a PA-Gd-BSA solution.

### Example 3

On the basis of the construction principle of the biomimetic nanomaterial described in FIG. 1, a protein and PA were connected using a bismuth ion with a high atomic number as a cation bridge, thereby forming a PA-bismuth-protein ternary complex nanoparticle.

A PA-Bi-BSA biomimetic nanomaterial was prepared according to the procedures in method II, with specific steps as follows: 125 µL of a 0.1 M PA solution was added to a reaction flask containing 9 ml of ddH₂O. The pH of the solution was adjusted to 7 with a 1 M NaOH solution. After adding 500 µL of a 1 mM BSA solution to the reaction flask, 375 µL of a 0.1 M BiCl₂ solution was added dropwise. The mixture was stirred at room temperature for reaction for 10 minutes. The solution was collected, and then centrifuged at 10,000 rpm for 20 minutes. The supernatant was transferred to an ultrafiltration tube and subjected to repeated ultrafiltration three times at 4000 rpm to remove impurities, thereby obtaining a PA-Bi-BSA solution.

### Example 4

(1) On the basis of the preparation principle diagram of FIG. 1, in this example, taking a paramagnetic manganese ion and a chemotherapeutic drug cisplatin as an example, a PA-Mn/Pt-BSA biomimetic nanomaterial was prepared according to the procedures of method I. Moreover, different molar ratios of PA : metal ion were set to screen for the preferred synthesis ratio. Specific steps were as follows: 125 µL of a 0.1 M PA solution was added to a reaction flask containing 9 ml of ddH₂O. According to different molar ratios of PA : Mn : Pt, including PA : Mn : Pt of 1 : 1 : 2, 1 : 1.5 : 1.5 and 1 : 2 : 1, 125 µL, 187.5 µL and 250 µL of a 0.1 M MnCl₂solution and 250 µL, 187.5 µL and 125 µL of a 0.1 M cis-diamminediaquaplatinum(II) solution were correspondingly added dropwise. The mixture was stirred uniformly. Then, 500 µL of a 1 mM BSA solution was added to the reaction flask, and the pH of the solution was quickly adjusted to 7 with a 1 M NaOH solution. The mixture was stirred at room temperature for 10 minutes. The solution was collected, and then centrifuged at 10,000 rpm for 20 minutes. The supernatant was transferred to an ultrafiltration tube and subjected to repeated ultrafiltration three times at 4000 rpm to remove impurities, thereby obtaining six PA-Mn/Pt-BSA solution samples.

### (2) Determination of the relaxivity of PA-Mn/Pt-BSA prepared in different molar ratios

Screening for the preferred metal ion ratio in PA-Mn/Pt-BSA was performed to meet the requirements for magnetic resonance imaging monitoring. Taking the above six prepared PA-Mn/Pt-BSA biomimetic nanomaterials with different ratios of PA : metal ion as examples, the relaxivity of each PA-Mn/Pt-BSA sample was determined to identify the preferred metal ion ratio of the constructed PA-based ternary complex biomimetic nanomaterials for magnetic resonance imaging.

The six PA-Mn/Pt-BSA biomimetic nanomaterial samples were placed on the sample holder of a 1.5 T nuclear magnetic resonance spectrometer for T1 value measurement. On the basis of T1 values of the samples with different ratios, linear regression was performed with concentration (C) as the abscissa and T1 value as the ordinate to construct a fitting curve, and then the longitudinal relaxivity (r1) values of the samples were calculated.

The results are shown in Table 3. Under the condition of ensuring a specific Mn ion loading efficiency, the PA-Mn/Pt-BSA nanoparticle prepared in a ratio of PA : Mn : Pt = 1 : 1 : 2 had a higher longitudinal relaxivity (11.88) compared to those prepared at other ratios. Therefore, the preferred molar ratio of PA : Mn : Pt in the PA-Mn/Pt-BSA ternary complex is 1 : 1 : 2.

**Table 3 Relaxivity of PA-Mn/Pt-BSA samples in different ratios**

| PA/Mn/Pt | r1 (mM⁻¹S⁻¹) | Mn (yield%) |
|---|---|---|
| 1 : 1 : 2 | 11.88 | 27.30 |
| 1 : 1.5 : 1.5 | 9.94 | 32.99 |
| 1 : 2 : 1 | 7.21 | 36.69 |

### Example 5

On the basis of the preparation principle diagram of FIG. 1, in this example, taking a paramagnetic manganese ion and a radiotherapeutic drug radium chloride as examples, a PA-Mn/²²³Ra-BSA biomimetic nanomaterial was prepared according to the procedure of method II. Specific steps were as follows: 125 µL of a 0.1 M PA solution was added to a reaction flask containing 9 ml of ddH₂O. The pH of the solution was adjusted to 7 with a 1 M NaOH solution. After adding 500 µL of a 1 mM BSA solution to the reaction flask, 375 µL of a 0.1 M MnCl₂ solution and 250 µL of a ²²³RaCl₂ solution with a radioactivity concentration of 1 Mkg/ml were added dropwise. The mixture was stirred at room temperature for reaction for 10 minutes. The solution was collected, and then centrifuged at 10,000 rpm for 20 minutes. The supernatant was transferred to an ultrafiltration tube and subjected to repeated ultrafiltration three times at 4000 rpm to remove impurities, thereby obtaining a PA-Mn/²²³Ra-BSA solution.

### Example 6

On the basis of the preparation principle diagram of FIG. 1, using polypeptide RGD as a carrier to load a paramagnetic manganese ion and a chemotherapeutic drug cisplatin (CDDP), a PA-Mn/Pt-RGD biomimetic nanomaterial was prepared according to method II. Specific steps were as follows: 125 µL of a 0.1 M PA solution was added to a reaction flask containing 9 ml of ddH₂O. The pH of the solution was adjusted to 10 with a 1 M NaOH solution. After adding 500 µL of a 1 mM RGD solution to the reaction flask, 125 µL of a 0.1 M MnCl₂ solution and 250 µL of a 0.1 M cis-diamminediaquaplatinum(II) solution were added dropwise. The mixture was stirred at room temperature for reaction for 10 minutes. The solution was collected, and then centrifuged at 10,000 rpm for 20 minutes. The supernatant was transferred to an ultrafiltration tube and subjected to repeated ultrafiltration three times at 4000 rpm to remove impurities, thereby obtaining a PA-Mn/Pt-RGD solution.

### Test example

### Characterization and in vitro/in vivo theranostic effect verification of the PA-based ternary complexes prepared in the present disclosure

### (1) Detection of particle size and morphology of the PA-based ternary complexes by transmission electron microscopy

Taking PA-Mn-BSA prepared in Example 1 with PA : Mn = 1 : 3, PA-Gd-BSA prepared in Example 2, and PA-Mn/Pt-BSA prepared in Example 4 with PA : Mn : Pt = 1 : 1 : 2 as examples, the morphology of the PA-based ternary complexes was observed using transmission electron microscopy. The solution was diluted to the appropriate concentration, dropped onto a carbon grid, and placed under a transmission electron microscope to observe the morphology and particle size of the above PA-Mn-BSA, PA-Gd-BSA and PA-Mn/Pt-BSA nanoparticles. The results are shown in FIG. 2, 3 and 4, respectively. All three PA-based ternary complex biomimetic nanomaterials had a small particle size of about 10 nm in diameter, with uniform size distribution and good dispersity.

### (2) Particle size monitoring of PA-based ternary complexes by dynamic light scattering

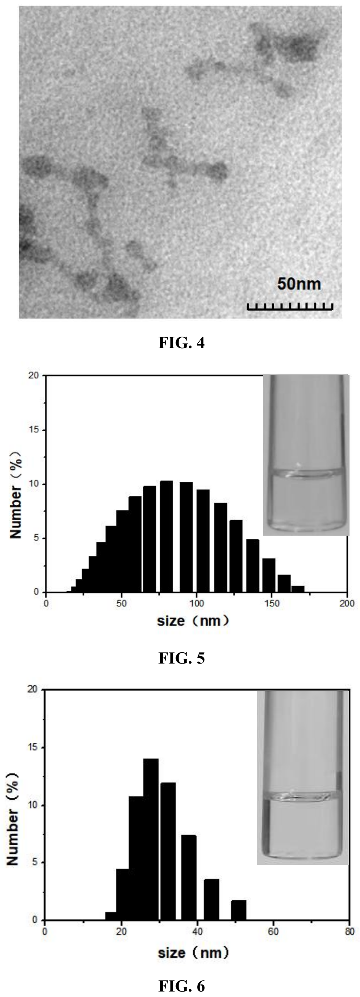

The hydrodynamic particle sizes of the PA-based ternary complex biomimetic nanomaterials PA-Bi-BSA, PA-Mn/²²³Ra-BSA and PA-Mn/Pt-RGD prepared in Examples 3, 5 and 6 were characterized by dynamic light scattering, to observe the particle sizes of the biomimetic nanomaterials. The solutions were diluted to an appropriate concentration and placed in a Malvern particle size analyzer to measure the hydrodynamic particle sizes of the biomimetic nanomaterials. The results are shown in FIG. 5, FIG. 6 and FIG. 7. The biomimetic nanomaterials PA-Bi-BSA, PA-Mn/²²³Ra-BSA and PA-Mn/Pt-RGD all formed clear and transparent solutions, indicating good solubility. Moreover, the particle sizes of all three biomimetic nanomaterials followed a normal distribution. The particle size of PA-Bi-BSA was about 80 nm, the particle size of PA-Mn/²²³Ra-BSA was about 27 nm, and the particle size of PA-Mn/Pt-RGD was about 8 nm. With the small particle sizes, all biomimetic nanomaterials meet the requirements for applications in bionanomedicine.

### (3) In vitro MRI effect

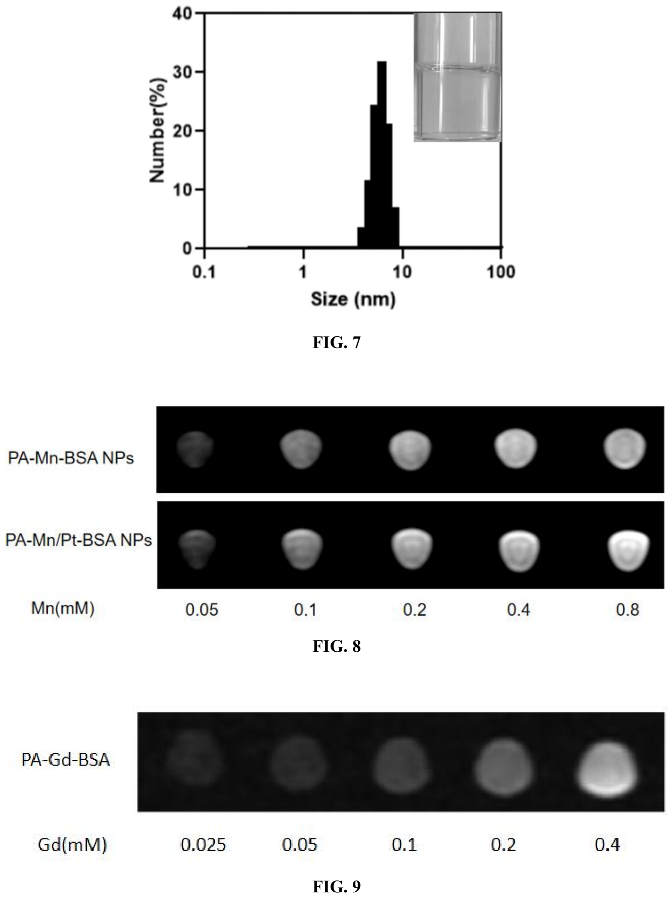

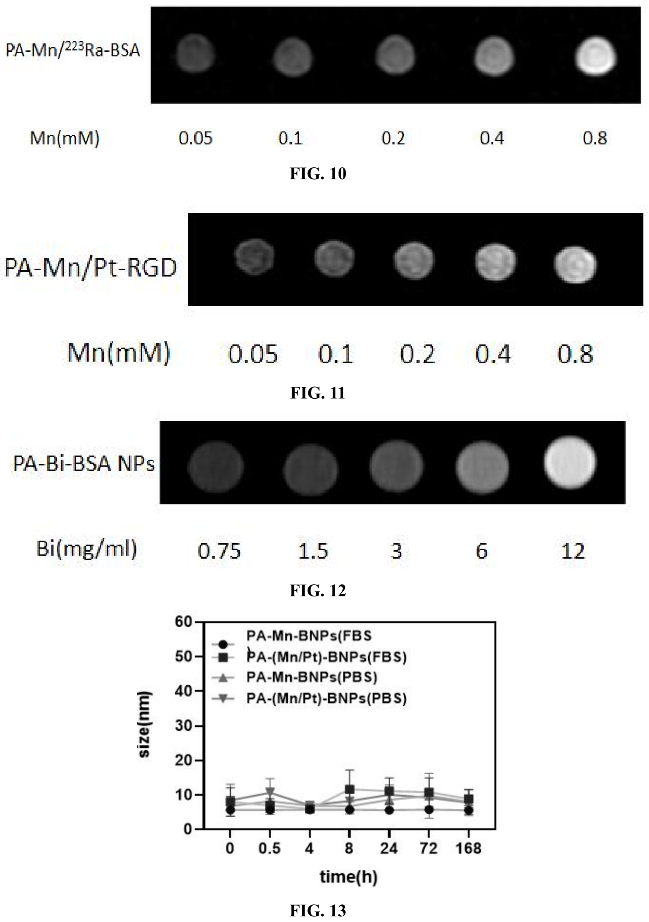

Taking PA-Mn-BSA (PA : Mn = 1 : 3), PA-Gd-BSA, PA-Mn/Pt-BSA (PA : Mn : Pt = 1 : 1 : 2), PA-Mn/²²³Ra-BSA and PA-Mn/Pt-RGD prepared in Examples 1, 2, 4, 5 and 6 as examples, the *in vitro* magnetic resonance imaging effects of monometallic or multimetallic PA-based ternary complexes were verified. The ternary complex solutions were diluted to different concentrations and subjected to T1WI imaging using a GE 3.0T magnetic resonance scanner. The results showed that as the concentration increased, the TIWI signals of PA-Mn-BSA (FIG. 8), PA-Mn/Pt-BSA (FIG. 8), PA-Gd-BSA (FIG. 9), PA-Mn/²²³Ra-BSA (FIG. 10) and PA-Mn/Pt-RGD (FIG. 11) solutions gradually increased, ultimately exhibiting extremely high brightness on T1WI images. Moreover, a comparison of the magnetic resonance images of PA-Mn-BSA and PA-Mn/Pt-BSA in FIG. 8 showed that the magnetic resonance imaging effects of PA-Mn-BSA and PA-Mn/Pt-BSA were substantially similar. This indicates that the doping of the therapeutic metal Pt into the Mn-based ternary complex has a negligible impact on the magnetic resonance imaging capability of Mn. The above results show that the PA-based ternary complex biomimetic nanomaterials prepared with either a single metal ion or multiple metal ions all exhibit superior T1 imaging effects *in vitro.*

### (4) In vitro CT imaging effect of PA-based ternary complexes

To verify that PA-based ternary complexes prepared with metal ions capable of imaging by alternative modalities also exhibit good imaging effects, such as bismuth, which has a high atomic number and thus enables CT imaging, PA-Bi-BSA prepared in Example 3 was taken as an example for validation. The PA-Bi-BSA solution was prepared at different concentrations and subjected to CT scans using a 64-slice CT scanner under the parameters: 100 kev, 90 ms. The results are shown in FIG. 12. As the concentration increased, the brightness of the PA-Bi-BSA solution gradually increased. At 12 mg/mL, the PA-Bi-BSA solution had high brightness and thus exhibited a good CT imaging effect. This indicates that the prepared PA-Bi-BSA solution possesses the potential to be used as a CT contrast agent, while also providing a reference for the preparation of PA-based ternary complex biomimetic nanomaterials using various other imaging metal ions.

### (5) Stability of PA-based ternary complex biomimetic nanomaterials

To verify the stability of the PA-based ternary complex biomimetic nanomaterials, PA-Mn-BSA (PA : Mn = 1 : 3) and PA-Mn/Pt-BSA (PA : Mn : Pt = 1 : 1 : 2) prepared in Examples 1 and 4 were taken as examples for the validation of particle size changes of the biomimetic nanomaterials in different environments (PBS and FBS). PA-Mn-BSA and PA-Mn/Pt-BSA nanoparticles were placed in PBS and FBS, respectively. The turbidity change of the solutions was observed at different time points (30 min, 4 h, 8 h, 1 d, 3 d, 7 d and 14 d), and the hydrodynamic particle sizes of the solutions were monitored by a dynamic light scattering particle size analyzer. The results are shown in FIG.13. The particle sizes of PA-Mn-BSA and PA-Mn/Pt-BSA nanoparticles did not change significantly during the 14-day monitoring period in PBS and FBS environments, indicating good stability.

### (6) pH-responsive reassembly performance of PA-based ternary complexes

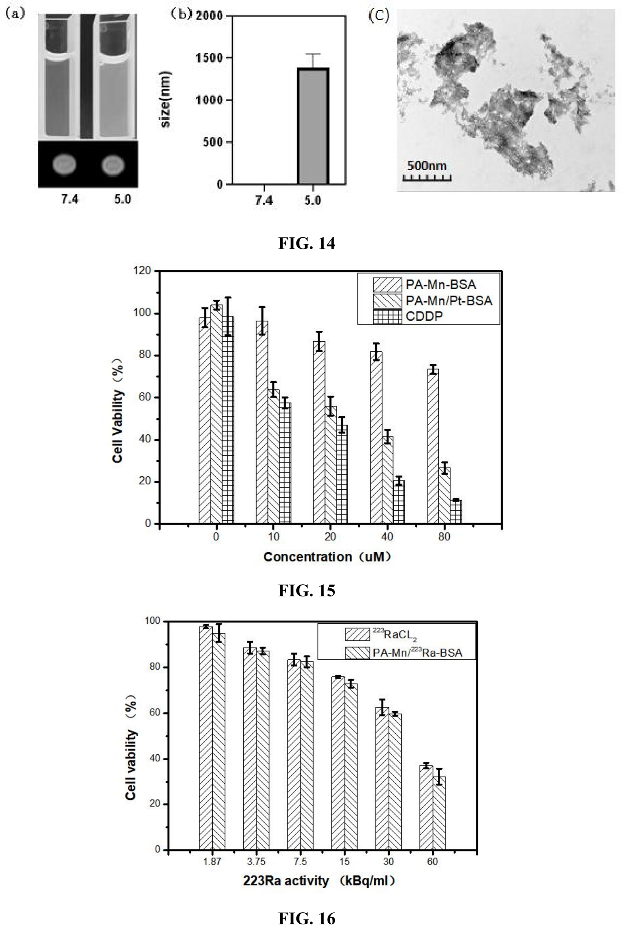

PA-Mn-BSA (PA : Mn = 1 : 3) prepared in Example 1 was taken as an example for validation. The pH values were adjusted to 7.4 and 5.0 with reference to the physiological pH of the human body (7.4) and the pH of tumor cell endosomes (5.0). The particle size changes of the nanoparticles under different pH conditions were observed using a particle size analyzer. The results showed that when the pH was adjusted from 7.4 to 5.0, the solution changed significantly from clear to turbid, as shown in (a) in FIG. 14, and the particle size of nanoparticles in the solution exhibited an explosive increase, as shown in (b) in FIG. 14. Moreover, the nanoparticles at pH 5.0 were observed by transmission electron microscopy, as shown in (c) in FIG. 14. Compared to PA-Mn-BSA (PA : Mn = 1 : 3) under physiological pH (FIG. 2), the ternary complex PA-Mn-BSA showed obvious aggregation at pH 5.0, demonstrating pH-responsive reassembly capability. This indicates that under the acidic conditions of tumors, the nanotheranostic probe can undergo pH-responsive aggregation, which prevents the probe from re-entering the blood circulation, thereby enhancing the accumulation and retention of the nanoparticles at the tumor site, achieving long-term effective retention at the tumor site, and enabling continuous imaging monitoring and therapy of the tumor.

### (7) Growth inhibition rate on tumor cells

### 1) Growth inhibition rates of PA-Mn-BSA and PA-Mn/Pt-BSA biomimetic nanomaterials on tumor cells.

This experiment was performed to verify *in vitro* that after loaded with chemotherapeutic metals via the ternary complex, the biomimetic nanomaterials can still maintain their antitumor activity and exert effective growth inhibitory effects on tumor cells. Using cisplatin as a control, PA-Mn-BSA (PA : Mn = 1 : 3) and PA-Mn/Pt-BSA (PA : Mn : Pt = 1 : 1 : 2) nanomaterials were co-incubated with PC-3 tumor cells for 72 h. The survival rate of the tumor cells was detected by the 3-(4,5)-dimethylthiahiazo (-z-y1)-3,5-di- phenytetrazoliumromide (MTT) assay. The results are shown in FIG. 15. Due to the intrinsic anticancer effect of PA, PA-Mn-BSA could inhibit the growth of tumor cells as the concentration increased, but the inhibitory effect was not significant. In contrast, the killing capacity of the PA-Mn/Pt-BSA group on the cells was close to that of the cisplatin group. This indicates that the PA-Mn/Pt-BSA biomimetic nanomaterial loaded with cisplatin retains the killing effect of cisplatin on tumor cells, providing a basis for their further use in *in vivo* tumor therapy and also supporting the feasibility of constructing multifunctional PA-based ternary complex biomimetic nanomaterials.

### 2) Growth inhibition rate of PA-Mn/²²³Ra-BSA biomimetic nanomaterial on tumor cells.

This experiment was performed to verify *in vitro* that after loaded with radiotherapeutic metals via the ternary complex, the biomimetic nanomaterials can still maintain their antitumor activity and exert effective growth inhibitory effects on tumor cells. Using ²²³RaCl₂ as a control, PA-Mn/²²³Ra-BSA prepared in Example 5 was co-incubated at different radioactivities with PC-3 tumor cells for 72 h. The survival rate of the tumor cells was detected by the MTT assay. The results are shown in FIG. 16. As the radioactivity increased, the proliferation of PC-3 cells treated with PA-Mn/²²³Ra-BSA was significantly inhibited, and the inhibitory effect of PA-Mn/²²³Ra-BSA on tumor cell growth was comparable to that of ²²³RaCl₂. This indicates that PA-Mn/²²³Ra-BSA loaded with the radioactive element ²²³Ra retains the killing effect of ²²³Ra on tumor cells, providing a basis for the construction of multifunctional PA-based ternary complex biomimetic nanomaterials.

### 3) Growth inhibition rate of PA-Mn/Pt-RGD biomimetic nanomaterial on tumor cells.

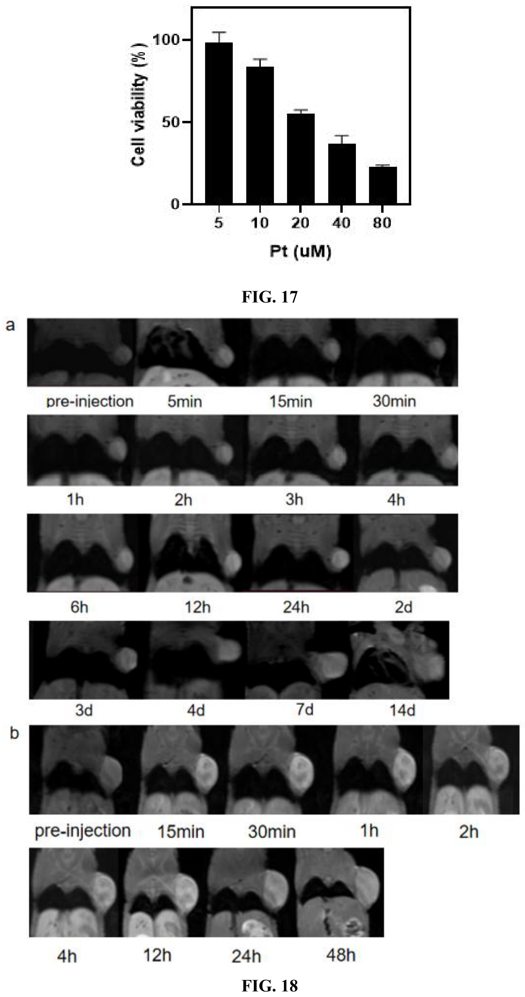

This experiment was performed to verify *in vitro* that the ternary complex biomimetic nanomaterials constructed with peptides as carriers can still maintain the antitumor activity of the loaded metal drugs and exert effective growth inhibitory effects on tumor cells. The PA-Mn/Pt-RGD biomimetic nanomaterial prepared in Example 6 was co-incubated at different concentrations with PC-3 tumor cells for 72 h. The survival rate of the tumor cells was detected by the MTT assay. The results are shown in FIG. 17. As the concentration of PA-Mn/Pt-RGD increased, the growth of tumor cells was significantly inhibited. This indicates that the PA-Mn/Pt-RGD biomimetic nanomaterial loaded with cisplatin retains the killing effect on tumor cells, providing a basis for the construction of multifunctional PA-based ternary complex biomimetic nanomaterials via polypeptides.

### (8) In vivo tumor imaging monitoring

### In vivo tumor imaging monitoring of PA-Mn-BSA and PA-Mn/Pt-BSA biomimetic nanomaterials

This experiment was performed to try to verify that the PA-based ternary complex biomimetic nanomaterials of the present disclosure can achieve excellent imaging effects *in vivo* when loaded with imaging metal ions. In this example, PA-Mn-BSA (PA : Mn = 1 : 3) and PA-Mn/Pt-BSA (PA : Mn : Pt = 1 : 1 : 2) prepared in Examples 1 and 4 were taken as examples for the validation of the *in vivo* magnetic resonance imaging effects thereof.

PA-Mn-BSA or PA-Mn/Pt-BSA solution was injected into PC-3 tumor-bearing mice via the tail vein, and continuous T1 magnetic resonance imaging monitoring of tumors was performed using a 3.0 T magnetic resonance scanner at different time points before and after injection. The results are shown in a in FIG. 18. After administration of PA-Mn-BSA, the T1WI signal at the tumor site of PC-3 tumor-bearing mice was significantly enhanced, with the enhancement effect continuously increasing and peaking at 12 hours. Moreover, the enhancement effect at the tumor site was maintained for a long time, and could still be observed on day 14. Meanwhile, b in FIG. 18 showed that after administration of PA-Mn/Pt-BSA, the T1WI signal at the tumor site of PC-3 tumor-bearing mice was also significantly enhanced, with the enhancement pattern similar to that of PA-Mn-BSA treatment and peaking at 12 hours. As time elapsed, the tumor signal began to decrease but still maintained an enhancement effect for a long time; this long-term enhancement effect suggests that the biomimetic nanomaterials can achieve long-term retention at the tumor site. The above results showed that both PA-Mn-BSA and PA-Mn/Pt-BSA biomimetic nanomaterials could achieve a good magnetic resonance imaging effect *in vivo,* and could be retained at the tumor site to achieve a long-term enhancement effect. This confirms that the PA-based ternary complex biomimetic nanomaterials of the present disclosure, after loaded with imaging metals, can realize imaging, diagnosis, and monitoring of tumors *in vivo.* Moreover, the biomimetic nanomaterials can achieve long-term retention at the tumor site and are expected to exert a long-term therapeutic effect on tumors, thereby enhancing the therapeutic effect of drugs.

### (9) In vivo tumor inhibitory effect

### 1) In vivo tumor inhibitory effect of PA-Mn/Pt-BSA biomimetic nanomaterial

This experiment was performed to verify that the biomimetic nanomaterials of the present disclosure can achieve tumor growth inhibition *in vivo* when chelated with chemotherapeutic metal ions. PA-Mn/Pt-BSA (PA : Mn : Pt = 1 : 1 : 2) prepared in Example 4 was taken as an example for the validation of the *in vivo* tumor inhibitory effect thereof.

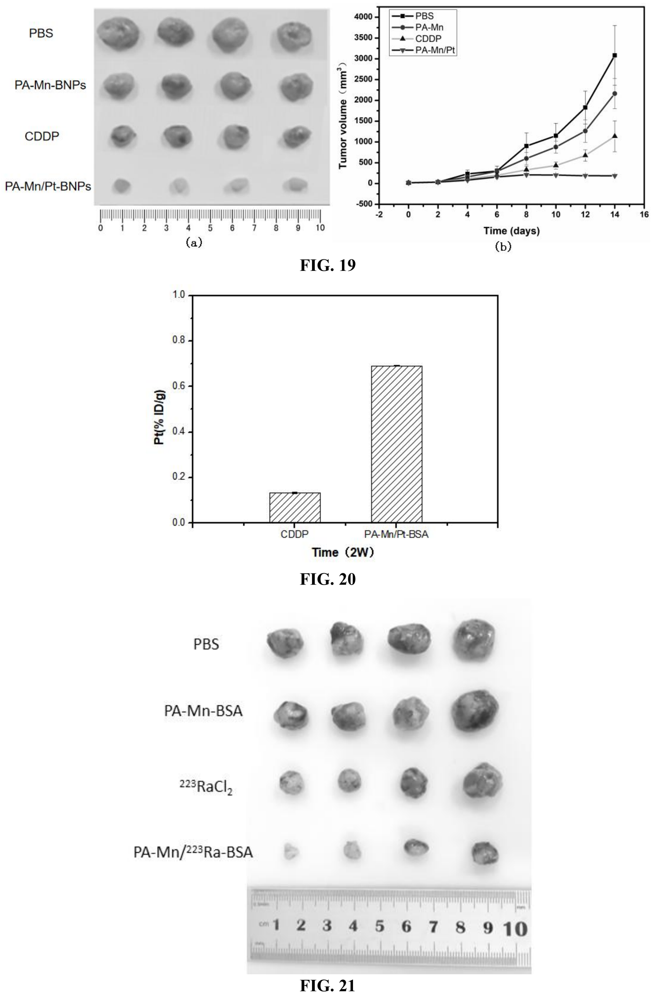

A PC-3 tumor-bearing mouse model was constructed. When the tumors grew to 100 mm³, PA-Mn/Pt-BSA solution was injected via the tail vein of the mice, with PBS, CDDP and PA-Mn-BSA (PA : Mn = 1 : 3) prepared in Example 1 as controls. Tumor size was measured every other day for 14 consecutive days after injection to observe the tumor growth inhibitory effect in different groups. Tumors were dissected and collected from the mice on day 14 for size observation. The results are shown in (a) in FIG. 19. Compared to the PBS, CDDP and PA-Mn-BSA groups, the PA-Mn/Pt-BSA group exhibited the smallest tumor volume, demonstrating a very strong tumor growth inhibitory effect. In contrast, the PA-Mn-BSA group showed no significant change in tumor volume compared to the PBS group, demonstrating an insignificant growth inhibitory effect. The CDDP group had a smaller tumor volume than the PBS group, but the tumor inhibitory effect thereof was still inferior to that of the PA-Mn/Pt-BSA group. Moreover, the tumor growth curve in (b) in FIG. 19 also showed that after treatment with PA-Mn/Pt-BSA, the tumor growth in the PA-Mn/Pt-BSA group was slow and significantly inhibited, compared to the exponential tumor growth in the PBS group. The above results indicate that the PA-Mn/Pt-BSA biomimetic nanomaterial chelating a therapeutic metal (cisplatin) has a significant growth inhibitory effect on tumors *in vivo.* Moreover, tumors from the CDDP group and the PA-Mn/Pt-BSA group were taken and detected for Pt content by ICP-MS on day 14. The results are shown in FIG. 20. The Pt content at the tumor site on day 14 was significantly higher in the PA-Mn/Pt-BSA group than in the CDDP group. This is consistent with the magnetic resonance imaging results of PA-Mn-BSA (PA : Mn = 1 : 3) and PA-Mn/Pt-BSA (PA : Mn : Pt = 1 : 1 : 2) prepared in Examples 1 and 4, indicating that PA-Mn/Pt-BSA can enable long-term accumulation of the chemotherapeutic agent cisplatin at the tumor site, thereby achieving a long-term chemotherapeutic effect.

### 2) In vivo tumor inhibitory effect of PA-Mn/²²³Ra-BSA

This experiment was performed to verify that the biomimetic nanomaterials of the present disclosure can achieve tumor growth inhibition *in vivo* when chelated with a radiotherapeutic metal ion. PA-Mn/²²³Ra-BSA prepared in Example 5 was taken as an example for the validation of the *in vivo* tumor inhibitory effect thereof.

A PC-3 tumor-bearing mouse model was constructed. When the tumors grew to 100 mm³, PA-Mn/²²³Ra-BSA solution was injected via the tail vein of the mice, with PBS, ²²³RaCl₂ and PA-Mn-BSA (PA : Mn = 1 : 3) prepared in Example 1 as controls. Tumor size was measured every other day for 10 consecutive days after injection to observe the tumor growth inhibitory effect in different groups. Tumors were dissected and collected from the mice on day 10 for size observation. The results are shown in FIG. 21. Compared to the PBS, ²²³RaCl₂ and PA-Mn-BSA groups, the PA-Mn/²²³Ra-BSA group exhibited the smallest tumor volume, demonstrating a very strong tumor growth inhibitory effect. The ²²³RaCl₂ group had a smaller tumor volume than the PBS group, but the tumor inhibitory effect thereof was still inferior to that of the PA-Mn/²²³Ra-BSA group.

### (10) Safety evaluation

Since most chemotherapeutic drugs have toxic side effects, the safety was evaluated using PA-Mn-BSA (PA : Mn = 1 : 3) and PA-Mn/Pt-BSA (PA : Mn : Pt = 1 : 1 : 2) prepared in Examples 1 and 4 as examples.

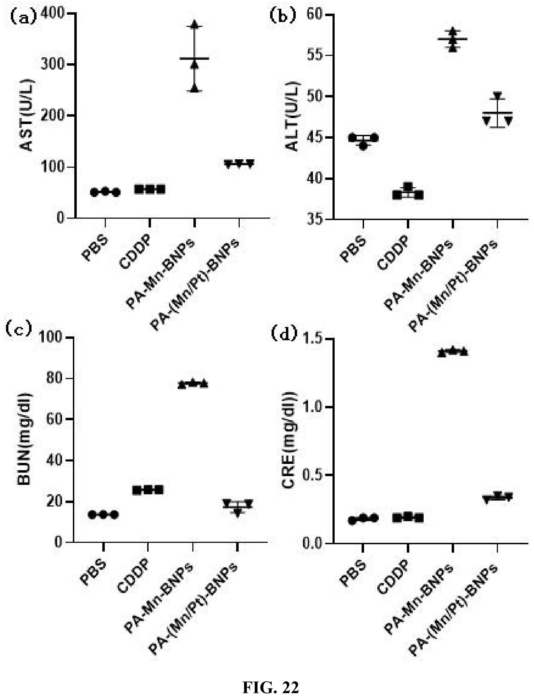

Normal Kunming mice were selected, and PBS, CDDP, PA-Mn-BSA and PA-Mn/Pt-BSA were separately injected via the tail vein of the mice. Blood biochemical analysis was performed 24 hours later. The results are shown in FIG. 22. After treatment with CDDP, the levels of aspartate transaminase (AST), alanine transaminase (ALT), blood urea nitrogen (BUN) and creatinine (CRE) in the mice were significantly higher than those in the PBS group, demonstrating obvious hepatotoxicity and nephrotoxicity. In contrast, after treatment with PA-Mn-BSA and PA-Mn/Pt-BSA, the blood biochemical indicators in the mice showed no significant changes compared with those in the PBS group, demonstrating no obvious hepatotoxicity or nephrotoxicity risks. Compared to the mice treated with free CDDP, the mice treated with cisplatin-loaded PA-Mn/Pt-BSA showed significantly reduced hepatotoxicity and nephrotoxicity. This suggests that the PA-based ternary complex biomimetic nanomaterials of the present disclosure can significantly reduce the toxic and side effects of the loaded drugs. The above results indicate that PA-Mn-BSA and PA-Mn/Pt-BSA have good biosafety, demonstrating that the biomimetic nanomaterials constructed from PA-based ternary complexes have high biocompatibility.

In summary, the present disclosure provides a preparation method for a PA-based ternary complex biomimetic nanomaterial. Based on this method, the defect of low solubility of phytates is addressed, and a soluble biomimetic nanomaterial is constructed to meet the application requirements of tumor diagnostic imaging, tumor therapy and integrated theranostics. The biomimetic nanomaterial prepared by the method has high biocompatibility and can be reassembled *in situ* in the acidic environment of tumors to achieve long-term retention at tumor sites, thereby realizing long-acting monitoring and therapeutic effects.

It is apparent to those skilled in the art that the present disclosure is not limited to the details of the above exemplary embodiments, and that the present disclosure can be implemented in other specific forms without departing from the spirit or basic features of the present disclosure. Therefore, from any perspective, the embodiments should be regarded as exemplary and non-limiting. With the motivation of the present disclosure, those of ordinary skill in the art may also make many changes without departing from the purpose of the present disclosure, and all such changes fall within the scope of protection of the present disclosure.

## Claims

1. A phytic acid (PA)-based ternary complex biomimetic nanomaterial, wherein
the biomimetic nanomaterial is a ternary complex composed of PA, a metal ion and a protein/polypeptide, wherein a molar ratio of the PA to the metal ion is 1 : (1-6), and a molar ratio of the protein/polypeptide to the PA is 1 : 25; the ternary complex is formed by connecting the protein/polypeptide with the PA using the metal ion as a cation bridge.

2. The PA-based ternary complex biomimetic nanomaterial according to claim 1, wherein the metal ion is a multivalent metal cation.

3. The PA-based ternary complex biomimetic nanomaterial according to claim 1, wherein the protein/polypeptide is a negatively charged protein/polypeptide at a pH value of 7-10.

4. The PA-based ternary complex biomimetic nanomaterial according to claim 2, wherein the multivalent metal cation comprises:
a multivalent metal cation selected from the group consisting of Cr³⁺, Co²⁺, Mn²⁺, Fe²⁺, Fe³⁺, Cu²⁺, Cu³⁺, La²⁺, Gd³⁺, Ce³⁺, Tb³⁺, Pr³⁺, Dy³⁺, Nd³⁺, Ho³⁺, Pm³⁺, Er³⁺, Sm³⁺, Tm³⁺, Eu³⁺, Yb³⁺, and Lu³⁺ provided that the nanomaterial is configured for use in preparing a contrast agent for magnetic resonance imaging (MRI);
a multivalent metal cation selected from the group consisting of Yb³⁺, Lu³⁺, Bi²⁺, Hf⁴⁺, Re⁴⁺, W⁴⁺ and Ta⁵⁺ provided that the nanomaterial is configured for use in preparing a contrast agent for preparing a contrast agent for computed tomography (CT) imaging;
a multivalent metal cation selected from the group consisting of ¹⁷⁷Lu³⁺, ⁶⁸Ga³⁺, ⁹⁹mTc⁷⁺, Sm³⁺, ¹¹¹In³⁺, and ⁶²Cu²⁺ provided that the nanomaterial is configured for use in preparing a contrast agent for preparing a contrast agent for radionuclide imaging;
a multivalent metal cation selected from the group consisting of Yb³⁺, Lu³⁺, Er³⁺, and Nd³⁺ provided that the nanomaterial is configured for use in preparing a contrast agent for preparing a contrast agent for optical imaging;
a multivalent metal cation selected from the group consisting of Pt²⁺, Ru³⁺, and Os²⁺ provided that the nanomaterial is configured for use in preparing a contrast agent for preparing a drug for chemotherapy;
a multivalent metal cation selected from the group consisting of ¹⁷⁷Lu³⁺, ²²³Ra²⁺, ⁸⁹Sr²⁺, ⁶⁰Co²⁺, ¹⁹²Ir³⁺, and ¹⁸²Ta⁵⁺ provided that the nanomaterial is configured for use in preparing a contrast agent for preparing a contrast agent for a drug for radiotherapy; and
a multivalent metal cation selected from the group consisting of Re⁴⁺, W⁴⁺, and Bi²⁺ provided that the nanomaterial is configured for use in preparing a drug for photothermal therapy.

5. A method for preparing the PA-based ternary complex biomimetic nanomaterial according to any one of claims 1 to 4, comprising steps of:
S1-1: mixing PA and a metal ion solution at a molar ratio of PA to metal ion of 1 : (1-6), and stirring until uniform to obtain mixed solution A;
S1-2: adding a protein/polypeptide solution to the mixed solution A at a molar ratio of protein/polypeptide to PA of 1 : 25, and adjusting a pH value of a resulting solution to 7-10 to obtain mixed solution B; and
S1-3: centrifuging mixed solution B, removeing a resulting pellet, and subjecting a resulting supernatant to repeated ultrafiltration/dialysis to obtain a ternary complex solution, which is the biomimetic nanomaterial.

6. A method for preparing the PA-based ternary complex biomimetic nanomaterial according to any one of claims 1 to 4, comprising steps of:
S2-1: providing a PA solution, adjusting a pH value of the PA solution to 7-10, adding a protein/polypeptide solution to the pH-adjusted PA solution at a molar ratio of protein/polypeptide to PA of 1 : 25, and stirring until uniform to obtain mixed solution C;
S2-2: adding a metal ion solution to the mixed solution C at a molar ratio of metal ion to PA of 3 : 1, and stirring until uniform to obtain mixed solution D, wherein the metal ion in the metal ion solution is a metal ion that produces a precipitate after mixed with the PA solution; and
S2-3: centrifuging the mixed solution D, remove a resulting pellet, and subjecting a resulting supernatant to repeated ultrafiltration/dialysis to obtain a ternary complex solution, which is the biomimetic nanomaterial.

7. The method for preparing the PA-based ternary complex biomimetic nanomaterial according to claim 6, wherein the metal ion is one or more of Gd³⁺, Bi²⁺ and ²²³Ra²⁺.

8. Use of a PA-based ternary complex biomimetic nanomaterial, wherein the biomimetic nanomaterial according to any one of claims 1 to 4 or a biomimetic nanomaterial prepared by the method according to any one of claims 5 to 7 is used for a contrast agent for one or more of imaging modalities comprising magnetic resonance imaging (MRI), computed CT imaging, radionuclide imaging and optical imaging, wherein the metal ion is a multivalent metal cation comprising:
a multivalent metal cation selected from the group consisting of Cr³⁺, Co²⁺, Mn²⁺, Fe²⁺, Fe³⁺, Cu²⁺, Cu³⁺, La²⁺, Gd³⁺, Ce³⁺, Tb³⁺, Pr³⁺, Dy³⁺, Nd³⁺, Ho³⁺, Pm³⁺, Er³⁺, Sm³⁺, Tm³⁺, Eu³⁺, Yb³⁺, and Lu³+ provided that the nanomaterial is configured for use in preparing a contrast agent for MRI;
a multivalent metal cation selected from the group consisting of Yb³⁺, Lu³⁺, Bi²⁺, Hf⁴⁺, Re⁴⁺, W⁴⁺, and Ta⁵⁺ provided that the nanomaterial is configured for use in preparing a contrast agent for CT imaging;
a multivalent metal cation selected from the group consisting of ¹⁷⁷Lu³⁺, ⁶⁸Ga³⁺, ⁹⁹mTc⁷⁺, Sm³⁺, ¹¹¹In³⁺, and ⁶²Cu²⁺ provided that the nanomaterial is configured for use in preparing a contrast agent for radionuclide imaging; and
a multivalent metal cation selected from the group consisting of Yb³⁺, Lu³⁺, Er³⁺, and Nd³⁺ provided that the nanomaterial is configured for use in preparing a contrast agent for optical imaging.

9. Use of a PA-based ternary complex biomimetic nanomaterial, wherein
the biomimetic nanomaterial according to any one of claims 1 to 4 or a biomimetic nanomaterial prepared by the method according to any one of claims 5 to 7 is used for preparing a drug for chemotherapy, radiotherapy or photothermal therapy, wherein the metal ion is a multivalent metal cation comprising:
a multivalent metal cation selected from the group consisting of Pt²⁺, Ru³⁺, and Os²⁺ provided that the nanomaterial is configured for use in preparing a drug for chemotherapy;
a multivalent metal cation selected from the group consisting of ¹⁷⁷Lu³⁺, ²²³Ra³⁺, ⁸⁹Sr²⁺, ⁶⁰Co²⁺, ¹⁹²Ir³⁺, and ¹⁸²Ta⁵⁺ provided that the nanomaterial is configured for use in preparing a drug for radiotherapy; and
a multivalent metal cation selected from the group consisting of Re⁴⁺, W⁴⁺, and Bi²⁺ provided that the nanomaterial is configured for use in preparing a drug for photothermal therapy.
